# EUROPÄISCHE PATENTSCHRIFT

(11) **EP 3 700 880 B1**
(45) Veröffentlichungstag und Bekanntmachung des Hinweises auf die Patenterteilung: **10.04.2024**
(21) Anmeldenummer: 18789174.2
(22) Anmeldetag: 25.10.2018
(51) Int. Cl.: C07C 11/08, C07C 7/163

(54) **VERFAHREN ZUR ENTFERNUNG VON DIENEN AUS EINEM C3- BIS C5-KOHLENWASSERSTOFFE ENTHALTENDEN STOFFSTROM DURCH SELEKTIVHYDRIERUNG**
METHOD FOR REMOVING DIENES FROM A MATERIAL STREAM CONTAINING C3 TO C5 HYDROCARBONS BY SELECTIVE HYDROGENATION
PROCÉDÉ D'ÉLIMINATION DE DIÈNES D'UN FLUX CONTENANT DES HYDROCARBURES EN C3-C5 PAR HYDROGÉNATION SÉLECTIVE

(30) Priorität: 25.10.2017 EP 17198380
(43) Veröffentlichungstag der Anmeldung: 02.09.2020
(73) Patentinhaber: BASF SE, 67056 Ludwigshafen am Rhein (DE)
(72) Erfinder: ISELBORN, Stefan, 67056 Ludwigshafen (DE); UFER, Andreas Joerg, 67056 Ludwigshafen (DE); KOETTER, Joachim, 04416 Markkleeberg (DE)
(74) Vertreter: BASF IP Association
(86) Internationale Anmeldenummer: PCT/EP2018/079250
(87) Internationale Veröffentlichungsnummer: WO 2019/081628

(56) Entgegenhaltungen:
- WO-A1-2014/209736
- US-A- 2 934 574
- US-A- 4 260 840

## Beschreibung

Die Erfindung geht aus von einem Verfahren zur Entfernung von Dienen aus einem C₃- bis C₅-Kohlenwasserstoffe enthaltenden Stoffstrom durch Selektivhydrierung.

Durch die Selektivhydrierung werden die Diene zu Alkenen mit einer Doppelbindung oder den entsprechenden Alkanen umgesetzt und so aus dem Stoffstrom entfernt.

Alkene mit einer Doppelbindung finden zum Beispiel Einsatz als Monomer oder Co-Monomer in der Herstellung von Polymeren. Hierbei ist Buten-1 eine für einen solchen Einsatz typische Verbindung.

In Stoffströmen, die die gewünschten Verbindungen enthalten, sind im Allgemeinen noch Nebenkomponenten enthalten, die zum Beispiel als unerwünschte Nebenprodukte bei der Herstellung entstehen. Ein Teil der Nebenprodukte kann durch übliche Reinigungsverfahren, beispielsweise destillative Trennverfahren aus dem Rohprodukt abgetrennt werden. Insbesondere bei Nebenkomponenten, deren Siedepunkt nahe am Siedepunkt des gewünschten Produktes liegt, ist dies jedoch nicht immer auf einfache Weise möglich. Insbesondere bei der Herstellung und Aufreinigung von Buten-1 sind im Allgemeinen noch Reste an Butadien enthalten. Dies hat jedoch den Nachteil, dass bei Einsatz des Buten-1 als Monomer in der Polymerherstellung bereits diese kleinen Anteile an Butadien zu Schwierigkeiten führen kann, da das Butadien die Vernetzung der Polymere beeinflusst.

Aufgrund des ähnlichen Siedepunktes erfolgt die Entfernung des Butadiens üblicherweise durch eine Selektivhydrierung. Hierdurch kann der Anteil an Butadien im Produktstrom weiter reduziert werden. Allerdings hat die Selektivhydrierung den Nachteil, dass neben dem durch die Hydrierung entstehenden Buten-1 auch Buten-2 und weitere unerwünschte Nebenprodukte entstehen können. Die Bildung dieser Nebenprodukte hat dann wiederum den Nachteil, dass gegebenenfalls die gewünschte Konzentration an Buten-1 im Produktstrom nicht erreicht wird.

Verfahren zur Entfernung von Butadien aus Buten-1 und Butadien enthaltenden Stoffströmen durch Selektivhydrierung sind zum Beispiel beschrieben in US 4,260,840, WO-A 2015/170282, WO-A 2006/124167, US 3,481,999 oder DE-A 33 01 164. Auch die US 4,260,840 oder US 2,934,574 haben Verfahren zur Selektivhydrierung zum Gegenstand. WO-A 2014/209736 beschreibt ein Verfahren zur Reduktion des Dien-Anteils durch stufenweise Wasserstoffzufuhr.

Zur Verringerung der Verluste am gewünschten Zielprodukt, dem Buten-1, ist aus DE-A 33 01 164 oder WO-A 2006/124167 bekannt, die Reaktion in Gegenwart geringer Mengen an Kohlenmonoxid durchzuführen. Die Verluste am gewünschten Zielprodukt können zum Beispiel dadurch entstehen, dass der für die Selektivhydrierung zugegebene Wasserstoff nicht nur mit dem Butadien reagiert, sondern auch mit dem Buten-1. Dies führt dazu, dass möglicherweise aufgrund der Selektivhydrierung und den dadurch entstehenden Nebenprodukten die Zielspezifikation für den Gehalt an Zielprodukt im Stoffstrom nicht mehr erhalten wird. Insbesondere bei einem geringen Gehalt an Dienen in einem Stoffstrom mit hoher Reinheit an Zielprodukt wird in der Regel durch die Selektivhydrierung ein so großer Anteil an Nebenprodukten erzeugt, dass die Konzentration an Zielprodukt nach der Selektivhydrierung nicht mehr den geforderten Spezifikationen entspricht.

Neben der Entfernung von Dienen durch Selektivhydrierung ist es auch bekannt, Diene durch Adsorptionsverfahren aus einem Stoffstrom zu entfernen. Hierzu werden zum Beispiel Molekularsiebe oder Zeolithe eingesetzt, an denen bevorzugt die zu entfernenden Substanzen adsorbieren. Derartige Adsorptionsverfahren sind beispielsweise aus US 4,567,309, US 2,882,243, US 3,992,471 oder DD-A 111 197 bekannt.

Auch diese Verfahren haben jedoch sämtlich zum Nachteil, dass es nicht möglich ist, sehr kleine Mengen an Dienen aus dem Stoffstrom zu entfernen.

Aufgabe der vorliegenden Erfindung war es daher, ein Verfahren bereitzustellen, mit dem sich Diene aus einem C₃- bis C₅-Kohlenwasserstoffe enthaltenden Stoffstrom entfernen lassen und bei dem die Konzentration der Wertprodukte innerhalb einer vorgegebenen Spezifikation gehalten wird.

Zur Entfernung von Dienen aus einem C₃- bis C₅-Kohlenwasserstoffe enthaltenden Stoffstrom durch Selektivhydrierung bei einem vorgegebenen Reaktionsdruck und einer vorgegebenen Reaktionstemperatur in Gegenwart eines Hydrierkatalysators werden der Reaktionsdruck und die Reaktionstemperatur am Reaktoreingang so geregelt, dass der Reaktionsdruck am Reaktoreingang maximal um 0,01 bar vom vorgegebenen Reaktionsdruck und die Reaktionstemperatur am Reaktoreingang um maximal 0,1 °C von der vorgegebenen Reaktionstemperatur abweichen und der Anteil an der Selektivhydrierung zugeführtem Wasserstoff im Bereich von 2 bis 20 Mol je Mol Dien, das in dem C₃- bis C₅-Kohlenwasserstoffe enthaltenden Stoffstrom enthalten ist, liegt.

Überraschenderweise hat sich gezeigt, dass bei einer Durchführung der Selektivhydrierung derart, dass der Reaktionsdruck am Reaktoreingang maximal um 0,01 bar vom vorgegebenen Reaktionsdruck und die Reaktionstemperatur am Reaktoreingang um maximal 0,1°C von der vorgegebenen Reaktionstemperatur abweichen, unerwünschte Nebenreaktionen und damit die Bildung von Nebenprodukten, die zu einer Abweichung von einer gewünschten Spezifikation hinsichtlich der Produktreinheit führen können, unterbunden oder reduziert werden können. Das erfindungsgemäße Verfahren erlaubt es somit, die Selektivhydrierung so durchzuführen, dass eine Überhydrierung, das heißt eine Hydrierung oder Isomerisierung des gewünschten Zielpro duktes, durch die der Gehalt an Zielprodukt im Stoffstrom abnimmt und damit gegebenenfalls die für die geforderte Spezifikation notwendige Konzentration an Zielprodukt nicht mehr erreicht wird, vermieden oder zumindest deutlich reduziert werden kann.

Zur Lösung der Aufgabe erfolgt die Regelung der Reaktionstemperatur und des Reaktionsdrucks durch ein Verfahren zur Entfernung von Dienen aus einem C₃- bis C₅-Kohlenwasserstoffe enthaltenden Stoffstrom durch Selektivhydrierung, das folgende Schritte umfasst:
(a) Verdampfen eines Teils eines flüssigen C₃- bis C₅-Kohlenwasserstoffe enthaltenden Stoffstroms, Mischen eines gasförmigen und eines flüssigen C₃- bis C₅-Kohlenwasserstoffe enthaltenden Stoffstroms oder Kondensieren eines Teils eines gasförmigen C₃- bis C₅-Kohlenwasserstoffe enthaltenden Stoffstroms, so dass nach der Verdampfung 2 bis 50 mol-%, bevorzugt 3 bis 30 mol-% und insbesondere 5 bis 25 mol-% des C₃- bis C₅-Kohlenwasserstoffe enthaltenden Stoffstrom in der Gasphase vorliegen,
(b) Zufuhr des teilverdampften C₃- bis C₅-Kohlenwasserstoffe enthaltenden Stoffstroms in eine einen Hydrierkatalysator enthaltende Reaktionsstufe und Zugabe von Wasserstoff in einem Anteil von 2 bis 20 Mol je Mol Dien, das in dem C₃- bis C₅-Kohlenwasserstoffe enthaltenden Stoffstrom enthalten ist, wobei die Zugabe des Wasserstoffs zusammen mit dem teilverdampften C₃- bis C₅-Kohlenwasserstoffe enthaltenden Stoffstrom erfolgen kann oder über eine separate Zugabestelle,
(c) Trennen von Gasphase und Flüssigphase und Entnahme der Flüssigphase als Produktstrom.

Durch das Verdampfen eines Teils des C₃- bis C₅-Kohlenwasserstoffe enthaltenden Stoffstroms oder alternativ durch das Aufteilen des C₃- bis C₅-Kohlenwasserstoffe enthaltenden Stoffstroms in zwei Stoffströme, Verdampfen eines der beiden Stoffströme und anschließendes Vermischen der beiden Stoffströme, das Mischen eines gasförmigen und eines flüssigen C₃- bis C₅-Kohlenwasserstoffe enthaltenden Stoffstroms oder das Kondensieren eines Teils des C₃- bis C₅-Kohlenwasserstoffe enthaltenden Stoffstroms so dass ein Stoffstrom erzeugt wird, bei dem 2 bis 50 mol-%, bevorzugt 3 bis 30 mol-% und insbesondere 5 bis 25 mol-% in der Gasphase vorliegen, ist es möglich, die sehr engen Prozessbedingungen, das heißt einen Reaktionsdruck am Reaktoreingang, der maximal 0,01 bar von einem vorgegebenen Reaktionsdruck abweicht und eine Reaktionstemperatur am Reaktoreingang, die maximal 0,1°C von einer vorgegebenen Reaktionstemperatur abweicht, einzuhalten, bei denen eine Selektivhydrierung ohne eine Überhydrierung durchgeführt werden kann. Hierdurch ist es möglich, die gewünschte Spezifikation hinsichtlich der Konzentration an Wertprodukten einzuhalten.

Anders als die direkte Steuerung der Temperatur oder des Drucks ermöglicht es die Verdampfung eines Teils des eingesetzten C₃- bis C₅-Kohlenwasserstoffe enthaltenden Stoffstroms, das Mischen eines gasförmigen und eines flüssigen C₃- bis C₅-Kohlenwasserstoffe enthaltenden Stoffstroms oder das Kondensieren eines Teils des C₃- bis C₅-Kohlenwasserstoffe enthaltenden Stoffstroms die Reaktionsbedingungen mit einer sehr viel einfacheren Steuerung innerhalb der erforderlichen Fenster zu halten. So führen zum Beispiel kleine Änderungen an der Größe des verdampften Teilstroms noch nicht unmittelbar zu Änderungen von Druck und Temperatur im Reaktor. Anders als die Fenster für Temperatur und Druck, innerhalb derer die gewünschte Produktspezifikation erzielt wird, ist das Fenster für die Teilverdampfung größer, so dass hier die notwendige Zeit für eine Korrektur der Parameter verbleibt und nicht bereits während der Korrektur der Parameter die gewünschte Produktspezifikation nicht mehr erreicht wird.

Bei Verdampfung eines Teils des C₃- bis C₅-Kohlenwasserstoffe enthaltenden Stoffstroms kann die Einstellung des gasförmigen Anteils am eingesetzten C₃- bis C₅-Kohlenwasserstoffe enthaltenden Stoffstrom entweder durch Regelung des zugeführten Wärmestroms erfolgen oder auch dadurch, dass der C₃- bis C₅-Kohlenwasserstoffe enthaltende Stoffstrom geteilt wird, ein Teil verdampft wird und anschließend die beiden Teilströme vermischt werden.

Eine weitere Möglichkeit zur Einstellung des gasförmigen Anteils des C₃- bis C₅-Kohlenwasserstoffe enthaltenden Stoffstroms in Schritt (a), so dass 2 bis 50 mol-%, bevorzugt 3 bis 30 mol-% und insbesondere 5 bis 25 mol-% des C₃- bis C₅-Kohlenwasserstoffe enthaltenden Stoffstroms in der Gasphase vorliegen, ist, dass ein Inertgas zugegeben wird. Durch die Zugabe des Inertgases wird der Partialdruck der einzelnen Komponenten im C₃- bis C₅-Kohlenwasserstoffe enthaltenden Stoffstrom gesenkt, so dass auf diese Weise die Verdampfungstemperatur und der verdampfte Teil des C₃- bis C₅-Kohlenwasserstoffe enthaltenden Stoffstroms eingestellt werden kann.

Das Inertgas kann dabei vor, während oder nach dem Verdampfen eines Teils eines flüssigen C₃- bis C₅-Kohlenwasserstoffe enthaltenden Stoffstroms, dem Mischen eines gasförmigen und eines flüssigen C₃- bis C₅-Kohlenwasserstoffe enthaltenden Stoffstroms oder dem Kondensieren eines Teils eines gasförmigen C₃- bis C₅-Kohlenwasserstoffe enthaltenden Stoffstroms in Schritt (a) zugegeben werden. Wenn ein gasförmiger und ein flüssiger C₃- bis C₅-Kohlenwasserstoffe enthaltender Stoffstrom gemischt werden, ist es möglich, das Inertgas nur dem flüssigen, nur dem gasförmigen oder auch beiden C₃- bis C₅-Kohlenwasserstoffe enthaltenden Stoffströmen zuzugeben.

Wenn die Zugabe des Inertgases während der Verdampfung, dem Mischen oder dem Kondensieren erfolgt, so ist es möglich, dieses zum Beispiel über einen separaten Zulauf in den Apparat, in dem die Verdampfung, das Mischen oder das Kondensieren durchgeführt werden, zuzugeben.

Geeignete Inertgase, die bei der Verdampfung zugegeben werden können, sind zum Beispiel ausgewählt aus der Gruppe bestehend aus Stickstoff, Methan, Kohlendioxid, Edelgasen und Mischungen daraus. Besonders bevorzugt als Inertgase sind Methan und Stickstoff.

Die Menge des zuzugebenden Anteils an Inertgas wird so geregelt, dass bei einem zu geringen Anteil an gasförmigem C₃- bis C₅-Kohlenwasserstoffe enthaltendem Stoffstrom die Menge an Inertgas erhöht wird und bei einem zu großen Anteil an gasförmigem C₃- bis C₅-Kohlenwasserstoffe enthaltendem Stoffstrom die Menge an Inertgas verringert wird.

Besonders bevorzugt ist es, wenn der eingesetzte C₃- bis C₅-Kohlenwasserstoffe enthaltende Stoffstrom flüssig ist und der gasförmige Anteil durch Verdampfung eines Teils des C₃- bis C₅-Kohlenwasserstoffe enthaltenden Stoffstroms erzielt wird.

Die Bestimmung des Anteils der Gasphase des C₃- bis C₅-Kohlenwasserstoffe enthaltenden Stoffstroms erfolgt zum Beispiel am Austritt aus dem Verdampfer. Hier ist es möglich, jeden geeigneten Sensor einzusetzen, mit dem die Menge der Gasphase bestimmt werden kann. Hierzu können zum Beispiel übliche Phasendetektoren eingesetzt werden. Alternativ ist es auch möglich, den Gasanteil über den zugeführten Wärmestrom im Verdampfer und die Stoffeigenschaften zu berechnen. Der zugeführte Wärmestrom ergibt sich zum Beispiel über die Temperaturdifferenz und den Massenstrom eines eingesetzten Wärmeträgers zu Erwärmung zwischen Zulauf und Ablauf oder auch über die Temperaturdifferenz zwischen mittlerer Wandtemperatur und Temperatur des zu verdampfenden C₃- bis C₅-Kohlenwasserstoffe enthaltenden Stoffstroms.

Für die Verdampfung kann entweder ein separater Verdampfer eingesetzt werden oder es wird als Verdampfer ein Verdampfungsteil im Reaktor für die Selektivhydrierung eingesetzt.

Wenn als Verdampfer ein separater Verdampfer eingesetzt wird, kann jeder beliebige, dem Fachmann bekannte Verdampfer eingesetzt werden, mit dem eine geregelte Verdampfung durchgeführt werden kann. Die Wärmezufuhr kann dabei entweder indirekt mit einem Wärmeträgermedium erfolgen oder alternativ durch Beheizung mit einem elektrischen Heizelement. Als Wärmeträgermedium lassen sich alle üblichen Wärmeträgermedien einsetzen, beispielsweise Wasserdampf oder Thermalöle. Geeignete Verdampfer sind zum Beispiel Rohrbündelwärmeübertrager, Fallfilmverdampfer oder Dünnschichtverdampfer. Bevorzugt ist es jedoch, den C₃-bis C₅-Kohlenwasserstoffe enthaltenden Stoffstrom bei erhöhtem Druck zu erwärmen und in den Reaktor, der bei niedrigerem Druck betrieben wird, zu entspannen. Hierzu kann zum Beispiel ein Entspannungsventil eingesetzt werden.

In einer alternativen Ausführungsform werden die Schritte (a) und (b) in einem Apparat mit Verdampfungsteil und Reaktionsteil mit den Hydrierkatalysator enthaltendem Katalysatorbett durchgeführt, wobei der Verdampfungsteil so angeordnet ist, dass der C₃- bis C₅-Kohlenwasserstoffe enthaltenden Stoffstrom vor Eintritt in das Katalysatorbett so weit verdampft ist, dass 2 bis 50 mol-%, bevorzugt 3 bis 30 mol-% und insbesondere 5 bis 25 mol-% des C₃-bis C₅-Kohlenwasserstoffe enthaltenden Stoffstrom in der Gasphase vorliegen.

Als Apparat mit Verdampfungsteil und Reaktionsteil kann zum Beispiel ein Apparat eingesetzt werden, der im unteren Teil einen Verdampfungsteil und im oberen Teil den Reaktionsteil aufweist. Der Verdampfungsteil ist zum Beispiel ein Rohrbündelverdampfer oder ein Abschnitt mit Doppelmantel, der von einem Wärmeträgermedium durchströmt werden kann. Alternativ ist es auch möglich, eine elektrische Beheizung vorzusehen. Oberhalb des Verdampfungsteils ist der Reaktionsteil angeordnet. Dieser umfasst vorzugsweise ein Festbett mit dem Hydrierkatalysator.

Alternativ zur Teilverdampfung des gesamten C₃- bis C₅-Kohlenwasserstoffe enthaltenden Stoffstroms ist es auch möglich, dass zum Verdampfen eines Teils des C₃- bis C₅-Kohlenwasserstoffe enthaltenden Stoffstroms in Schritt (a) der C₃- bis C₅-Kohlenwasserstoffe enthaltende Stoffstrom in zwei Ströme geteilt wird, einer der zwei Ströme vollständig verdampft wird und nach dem Verdampfen wieder mit dem zweiten der zwei Ströme vermischt wird.

Durch das Teilen des C₃- bis C₅-Kohlenwasserstoffe enthaltenden Stoffstroms in zwei Teile, die vollständige Verdampfung eines Teils und die anschließende Vermischung der beiden Ströme wird der Anteil des verdampften Teils durch die Größe des zu verdampfenden Teilstroms festgelegt. Dies hat den Vorteil, dass ein Teilstrom vollständig verdampft wird und daher nicht bei der Verdampfung darauf geachtet werden muss, dass nur so viel Wärme zugeführt wird, dass der geforderte Anteil des Stoffstroms verdampft. Es muss somit nur darauf geachtet werden, den C₃- bis C₅-Kohlenwasserstoffe enthaltenden Stoffstrom in Stoffströme der erforderlichen Größe zu teilen. Da der eine Strom vollständig verdampft wird, muss hier nur darauf geachtet werden, dass der Strom auch vollständig und nicht nur teilweise verdampft ist. Um zu verhindern, dass der verdampfte Teilstrom nach der Vermischung mit dem nicht verdampften Strom unmittelbar wieder kondensiert, ist es weiterhin bevorzugt, wenn der zweite Teilstrom, der nicht verdampft wird, auf eine Temperatur knapp unterhalb, vorzugsweise 1 bis 10 °C unterhalb der Verdampfungstemperatur des C₃- bis C₅-Kohlenwasserstoffe enthaltenden Stoffstroms erwärmt wird. Alternativ ist es notwendig, den zu verdampfenden Stoffstrom so groß zu wählen, dass nach Vermischung und Kondensation noch eine ausreichend große Gasphase vorliegt.

Das Verdampfen des Teilstroms kann in jedem beliebigen Verdampfer durchgeführt werden. Geeignete Verdampfer entsprechen den vorstehend für die Ausführungsform, bei der der gesamte C₃- bis C₅-Kohlenwasserstoffe enthaltende Stoffstrom dem Verdampfer zugeführt wird.

Zur Einstellung des verdampften Anteils im C₃- bis C₅-Kohlenwasserstoffe enthaltenden Stoffstrom ist es bei Verdampfung in einem separaten Verdampfer weiterhin möglich, dem Verdampfer einen Phasenscheider nachzuschalten. Im Phasenscheider werden die Gasphase und die Flüssigphase voneinander getrennt und separat entnommen. Der Anteil der Gasphase im C₃-bis C₅-Kohlenwasserstoffe enthaltenden Stoffstrom kann dann durch Vermischung entsprechender Anteile an Gasphase und Flüssigphase erfolgen. Sollte der Anteil der Flüssigphase zu groß sein, wird ein Teil der Flüssigphase entnommen und zurück in den Verdampfer geleitet. Entsprechend wird bei einem zu großen Teil der Gasphase ein Teil der Gasphase entnommen und zurückgeführt. Auf diese Weise lassen sich die Anteile der Stoffströme in der Gasphase und in der Flüssigkeit so einstellen und vermischen, dass im gemischten Stoffstrom, der dann dem Reaktor zugeführt wird, der Anteil der Gasphase im Bereich von 2 bis 50 mol-%, bevorzugt 3 bis 30 mol-% und insbesondere 5 bis 25 mol-% liegt.

Insbesondere dann, wenn die Verdampfung in einem separaten Verdampfer durchgeführt wird und nicht ein Apparat eingesetzt wird, der einen Verdampfungsteil und einen Reaktionsteil umfasst, das heißt sowohl bei der Ausführungsform, bei der der gesamte C₃- bis C₅-Kohlenwasserstoffe enthaltende Stoffstrom einem Verdampfer zugeführt wird oder alternativ bei der Ausführungsform, bei der der C₃- bis C₅-Kohlenwasserstoffe enthaltende Stoffstrom geteilt und ein Teil verdampft wird, kann ein weiterer Teil des C₃- bis C₅-Kohlenwasserstoffe enthaltenden Stoffstroms beim Eintritt in den Reaktor verdampfen. Dies insbesondere dann, wenn der Druck im Reaktor niedriger ist als der Druck des Stoffstroms in der Leitung vor Eintritt in den Reaktor.

Wenn ein Teil des Stoffstroms bei Eintritt in den Reaktor verdampft, das heißt, wenn die Reaktionsstufe einen separaten Reaktor umfasst, wird die Verdampfung in Schritt (a) so gesteuert, dass beim Eintritt des C₃- bis C₅-Kohlenwasserstoffe enthaltenden Stoffstroms in den Reaktor ein weiterer Teil verdampft, so dass nach Eintritt des C₃- bis C₅-Kohlenwasserstoffe enthaltenden Stoffstroms in den Reaktor 2 bis 50 mol-%, bevorzugt 3 bis 30 mol-% und insbesondere 5 bis 25 mol-% des C₃- bis C₅-Kohlenwasserstoffe enthaltenden Stoffstroms in der Gasphase vorliegen.

Besonders bevorzugt ist es, dass die Verdampfung des Teils des C₃- bis C₅-Kohlenwasserstoffe enthaltenden Stoffstroms vollständig beim Eintritt in den Reaktor erfolgt. In diesem Fall wird dem Reaktor kein Verdampfer vorgeschaltet sondern ein Wärmeübertrager, in dem der C₃- bis C₅-Kohlenwasserstoffe enthaltende Stoffstrom erwärmt wird, wobei die Erwärmung auf eine Temperatur erfolgt, die unterhalb der Siedetemperatur liegt. Der Druck, bei dem der C₃- bis C₅-Kohlenwasserstoffe enthaltende Stoffstrom erwärmt wird, liegt oberhalb des Drucks im Reaktor. Derartige Wärmeübertrager werden auch als Zwangsumlaufentspannungsverdampfer bezeichnet.

Als Reaktor für die Selektivhydrierung kann jeder beliebige, dem Fachmann bekannte Reaktor eingesetzt werden, der sich für Selektivhydrierungen eignet. Derartige Reaktoren sind zum Beispiel Festbettreaktoren, in denen der Hydrierkatalysator als Festbett eingebracht ist. Besonders bevorzugt werden dabei Festbettreaktoren eingesetzt, die von oben mit den Edukten beschickt werden, sogenannte "Trickle-Flow-Reaktoren".

Geeignete Hydrierkatalysatoren sind zum Beispiel solche, die mindestens ein Metall der VIII. Gruppe des Periodensystems der Elemente (Fe, Ru, Os, Co, Rh, Ir, Ni, Pd, Pt), insbesondere Palladium, als Hydriermetall und gegebenenfalls zusätzlich einen Promotor auf einem oxidischen Träger umfassen.

Bevorzugt weist der Katalysator einen Durchmesser von 1,5 bis 10 mm, mehr bevorzugt 1,5 bis 5 mm und insbesondere 2,5 bis 3,5 mm, auf.

Vorzugsweise wird ein Katalysator eingesetzt, bei dem das Metall der VIII. Gruppe des Periodensystems der Elemente eine Schalenstruktur in dem Katalysator ausbildet.

Die Bezeichnung der Gruppen des Periodensystems der Elemente erfolgt gemäß der CAS-Nomenklatur (chemical abstracts service).

Der Katalysator kann darüber hinaus mindestens einen Promotor enthalten. Beispielsweise kann es sich hierbei um weitere Metalle der VIII., IB. und IIB. Gruppe des Periodensystems der Elemente handeln (Cu, Ag, Au, Zn, Cd, Hg). In einer bevorzugten Ausführungsform enthält der Katalysator neben dem Metall der VIII. Gruppe des Periodensystems der Elemente noch mindestens ein Metall aus der IB. Gruppe des Periodensystems der Elemente. Besonders bevorzugt ist hierbei Silber.

In einer besonders bevorzugten Ausführungsform enthält der erfindungsgemäße Katalysator Palladium und Silber.

Der Katalysator kann beliebige Formen, beispielsweise Stränge, Hohlstränge, Tabletten, Ringe, sphärische Teilchen oder Kugeln, aufweisen. Bevorzugt ist es, wenn der Katalysator als Strang ausgebildet ist.

Die Metalle können in reiner metallischer Form vorliegen, aber auch in Form von Verbindungen, beispielsweise in Form von Metalloxiden. Unter den Betriebsbedingungen eines Hydrierverfahrens liegen sie im Allgemeinen in Form von Metallen vor. Die Umwandlung etwaiger Oxide in Metalle kann auf dem Fachmann bekannte Weise vor dem Einsatz des Katalysators in einem Hydrierverfahren in oder außerhalb eines Hydrierreaktors, beispielsweise durch Vorreduzierung und, falls für Manipulationen mit dem vorreduzierten Katalysator erforderlich oder vorteilhaft, anschließende oberflächliche Passivierung erfolgen.

Der Gehalt des Katalysators an Metall oder Metallen der VIII. Gruppe des Periodensystems, insbesondere Palladium, beträgt vorzugsweise mindestens 0,01 Gew.-%, besonders bevorzugt mindestens 0,1 Gew.-%, insbesondere mindestens 0,15 Gew.-%. Vorzugsweise liegt dieser Gehalt bei höchstens 5 Gew.-%, besonders bevorzugt höchstens 1 Gew.-%, insbesondere höchstens 0,6 Gew.-%. Niedrigere und höhere Gehalte sind zwar möglich, aber im Normalfall wegen zu niedriger Aktivität oder zu hohen Rohstoffkosten wirtschaftlich unbefriedigend. In einer besonders bevorzugten Ausführungsform wird nur ein Hydriermetall, insbesondere Palladium, verwendet.

Das Verhältnis der Mengen von Hydriermetall der VIII. Gruppe des Periodensystems der Elemente und Zusatz- oder Dotierstoffen ist ein im Einzelfall zu optimierender Parameter. Vorzugsweise beträgt das Atomverhältnis von Metall der VIII. Gruppe des Periodensystems der Elemente, besonders bevorzugt Palladium, zu dem Promotor, besonders bevorzugt Silber, vorzugsweise 0,1 - 10, besonders bevorzugt 2 - 7, insbesondere 2,5 - 6.

Der oxidische Träger des Hydrierkatalysators ist bevorzugt Aluminiumoxid, besonders bevorzugt in einer Mischung aus δ-, θ- und α-Aluminiumoxid. Der Träger kann neben unvermeidbaren Verunreinigungen in gewissem Umfang auch andere Zusätze enthalten. Beispielsweise können andere anorganische Oxide wie Oxide von Metallen der IIA., IIIB., IVB., IIIA. und IVA. Gruppe des Periodensystems der Elemente enthalten sein, insbesondere Siliziumdioxid, Titandioxid, Zirkondioxid, Zinkoxid, Magnesiumoxid, Natriumoxid und Kalziumoxid. Der maximale Gehalt des Trägers an solchen von Aluminiumoxid verschiedenen Oxiden ist vom tatsächlich vorhandenen Oxid abhängig, aber im Einzelfall anhand des Röntgenbeugungsdiagramms des Hydrierkatalysators zu ermitteln, da eine Änderung der Struktur mit einer signifikanten Änderung des Röntgenbeugungsdiagramms einhergeht. Im Allgemeinen liegt der Gehalt an solchen, von Aluminiumoxid verschiedenen Oxiden, unterhalb von 50 Gew.-%, vorzugsweise unterhalb von 30 Gew.-%, besonders bevorzugt unterhalb von 10 Gew.-%. Der Reinheitsgrad des Aluminiumoxids liegt vorzugsweise höher als 99%.

Entsprechende Katalysatoren und geeignete Verfahren zu deren Herstellung sind zum Beispiel in der WO-A 2006/040159 oder in der WO-A 2006/040160 beschrieben.

Neben dem C₃- bis C₅-Kohlenwasserstoffe enthaltenden Stoffstrom wird dem Reaktor für die Selektivhydrierung Wasserstoff zugeführt. Die Menge an Wasserstoff liegt erfindungsgemäß im Bereich von von 2 bis 20 Mol je Mol Dien, das in dem C₃- bis C₅-Kohlenwasserstoffe enthaltenden Stoffstrom enthalten ist. Die Zugabe des Wasserstoffs kann entweder direkt in den Hydrierreaktor erfolgen oder alternativ in den C₃- bis C₅-Kohlenwasserstoffe enthaltenden Stoffstrom. Die Zugabe kann in diesem Fall entweder vor der Verdampfung, dem Mischen oder der Kondensation in Schritt (a) erfolgen oder alternativ nach der Verdampfung, dem Mischen oder dem Kondensieren. Wenn der Wasserstoff dem C₃- bis C₅-Kohlenwasserstoffe enthaltenden Stoffstrom zugegeben wird, wirkt dieser ebenfalls als Gas, mit dem der Anteil des gasförmigen Anteils des C₃- bis C₅-Kohlenwasserstoffe enthaltenden Stoffstroms eingestellt werden kann.

Der Druck im Hydrierreaktor, bei dem die Selektivhydrierung durchgeführt wird, liegt vorzugsweise im Bereich von 2 bis 20 bar, bevorzugt bei 2,2 bis 7 bar, und die Eintrittstemperatur des C₃- bis C₅-Kohlenwasserstoffe enthaltenden Stoffstroms bei 0 bis 80 °C, bevorzugt bei 20 bis 60°C. Wenn der C₃- bis C₅-Kohlenwasserstoffe enthaltende Stoffstrom bei der Zugabe in den Hydrierreaktor zur Verdampfung entspannt wird, wird die Erwärmung des C₃- bis C₅-Kohlenwasserstoffe enthaltenden Stoffstroms vorzugsweise bei einem Druck durchgeführt, der 0,5 bis 8 bar, mehr bevorzugt im Bereich von 0,5 bis 5 bar und insbesondere im Bereich von 0,5 bis 3,5 bar oberhalb des Drucks im Hydrierreaktor liegt.

Bevorzugt wird die Selektivhydrierung bei einem Druck im Bereich von 2 bis 20 bar und insbesondere bei einem Druck im Bereich von 2 bis 12 bar und einer Eintrittstemperatur des C₃- bis C₅-Kohlenwasserstoffe enthaltenden Stoffstroms im Bereich von 0 bis 80 °C und insbesondere im Bereich von 20 bis 60 °C durchgeführt.

In der Regel entsteht bei der Selektivhydrierung eine Flüssigphase als Produktstrom, die die C₃-bis C₅-Alkene mit genau einer Doppelbindung in der gewünschten Konzentration enthält. Zusätzlich können jedoch auch in der Gasphase C₃- bis C₅-Alkene mit genau einer Doppelbindung enthalten sein. Um auch diese als Produktstrom zu erhalten ist es bevorzugt, die Gasphase abzukühlen, so dass die in der Gasphase enthaltenen C₃- bis C₅-Alkene mit genau einer Doppelbindung kondensieren und ebenfalls als Produkt entnommen werden können.

Das erfindungsgemäße Verfahren eignet sich insbesondere zur Entfernung von Dienen aus C₃-bis C₅-Kohlenwasserstoffe enthaltenden Stoffströmen, die bereits einen hohen Anteil an C₃- bis C₅-Alkenen mit genau einer Doppelbindung enthalten und bei denen zur Einhaltung einer festgelegten Spezifikation der Anteil an C₃- bis C₅-Alkenen mit genau einer Doppelbindung nicht unter eine vorgegebene Konzentration fallen darf. Insbesondere eignet sich das Verfahren zur Entfernung von Dienen aus C₃- bis C₅-Kohlenwasserstoffe enthaltenden Stoffströmen, die mindestens 99 Gew.-% C₃- bis C₅-Alkene mit genau einer Doppelbindung enthalten.

Bei üblichen Verfahren zur Selektivhydrierung ist insbesondere bei Entfernung von Dienen aus einem Stoffstrom mit einem entsprechend hohen Anteil an Alkenen mit genau einer Doppelbindung nicht auszuschließen, dass auch der Gehalt an gewünschten Alkenen mit genau einer Doppelbindung durch Nebenreaktionen, zum Beispiel Überhydrierungen oder Isomerisierungen, reduziert wird, so dass im Produktstrom nach der Selektivhydrierung der Anteil an C₃- bis C₅-Alkenen mit genau einer Doppelbindung nicht mehr der vorgegebenen Spezifikation entspricht sondern niedriger liegt. Durch das erfindungsgemäße Verfahren ist es möglich, die Selektivhydrierung eines mindestens 99 Gew.-% C₃- bis C₅-Alkene mit genau einer Doppelbindung enthalten Stoffstroms so durchzuführen, dass auch der Produktstrom mindestens 99 Gew.-% C₃- bis C₅-Alkene mit genau einer Doppelbindung enthält.

In einer besonders bevorzugten Ausführungsform ist das C₃- bis C₅-Alken mit genau einer Doppelbindung ein Buten und insbesondere Buten-1. Entsprechend handelt es sich in diesem Fall bei dem Dien um Butadien und gegebenenfalls Pentadien, insbesondere um Butadien. Wenn das gewünschte Alken mit genau einer Doppelbindung Buten-1 ist, sind die unerwünschten Nebenprodukte, die in diesem Fall entstehen können, insbesondere Buten-2 und n-Butan.

Der C₃- bis C₅-Kohlenwasserstoffe enthaltende Stoffstrom enthält im Allgemeinen neben dem Dien noch weitere Komponenten als Verunreinigungen, im allgemeinen Kohlenwasserstoffe mit einer Anzahl an C-Atomen, die größer oder kleiner ist als die gewünschte, das heißt bei einem C₃- bis C₅-Kohlenwasserstoffe als Zielprodukt enthaltenden Stoffstrom zum Beispiel C₂-Kohlenwasserstoffe oder auch Kohlenwasserstoffe mit mehr als 5 Kohlenstoffen, zum Beispiel C₆- bis C₈-Kohlenwasserstoffe.

Wenn das Zielprodukt ein Buten ist, insbesondere Buten-1, können als Nebenkomponenten im Stoffstrom zum Beispiel auch C₃- oder C₅-Kohlenwasserstoffe enthalten sein.

Bei einem Einsatz des Butens als (Co-)Monomer in einer Polymerisationsreaktion sind von den Nebenprodukten jedoch insbesondere die Diene, insbesondere das Butadien, problematisch, da dieses die Vernetzung der Polymere beeinflusst. Aus diesem Grund ist es notwendig, das Butadien aus dem Stoffstrom zu entfernen, ohne die für die Polymerisation vorgegebene Spezifikation, das heißt den Gehalt an Buten, insbesondere Buten-1, zu verlassen. Das bedeutet, dass bei der Selektivhydrierung nicht oder nur in sehr geringem Umfang überhydriert oder isomerisiert werden darf. Insbesondere bei Buten-1 als Zielprodukt ist Butadien zu entfernen ohne den Gehalt an Buten-2 bzw. Butan (z. B. durch Isomerisierung bzw. Überhydrierung von Buten-1) auf Werte oberhalb der spezifizierten Grenzwerte zu erhöhen.

Das erfindungsgemäße Verfahren eignet sich insbesondere auch, aus einem bereits aufgearbeiteten C3- bis C5-Kohlenwasserstoffe enthaltenden Stoffstrom, der noch Spuren an Dienen enthält, den Anteil der Diene weiter zu reduzieren. Bei einem solchen Stoffstrom ist der Anteil der Diene im Allgemeinen kleiner als 1000 ppm. Insbesondere eignet sich das Verfahren zur Entfernung von Dienen durch Selektivhydrierung aus einem C3- bis C5-Kohlenwasserstoffe enthaltenden Stoffstrom, bei dem der Anteil an Dienen kleiner als 500 ppm und insbesondere kleiner als 200 ppm ist.

### Beispiele

In einem Rieselbettreaktor mit 200 ml eines Palladium/Silber-Katalysators, erhältlich zum Beispiel als H0-43 der BASF SE, als Hydrierkatalysator wurden 2,5 kg/h eines Stoffstroms mit einem Gehalt von 99,84 Buten-1 und 121 ppm beziehungsweise 182 ppm Butadien hydriert. Vor der Zugabe des Stoffstroms in den Rieselbettreaktor wurde der Stoffstrom in einem elektrisch betriebenen Verdampfer erhitzt und teilweise verdampft. Die Wärmezufuhr wurde so eingestellt, dass nach der Verdampfung etwa 15 Gew.-% des eingesetzten Stoffstroms in der Gasphase vorlagen.

Die Reaktion wurde so durchgeführt, dass die Temperatur am Reaktoreingang bei 40°C oder alternativ bei 30°C lag. Die Temperatur am Reaktoreingang wurde über den Druck am Reaktorausgang eingestellt.

Die Zusammensetzung des bei der Selektivhydrierung erhaltenen Produktstroms, der Anteil an zugeführtem Wasserstoff, die Temperatur am Reaktoreintritt und der Druck am Reaktoraustritt können den nachfolgenden Tabellen entnommen werden. Tabelle 1 führt die Prozessparameter für die einzelnen Beispiele auf und Tabelle 2 kann die jeweilige Zusammensetzung des dem Rieselbettreaktor entnommenen Produktstroms entnommen werden.

Für die Beispiele 1 und 2 wurde ein Feedstrom eingesetzt, der 99,84 Gew.-% Buten-1, 370 ppm n-Butan, 92 ppm trans-2-Buten, 112 ppm cis-2-Buten und 121 ppm Butadien enthielt, für die Beispiele 3, 4 und 5 wurde ein Feedstrom mit einem Gehalt von 99,84 Gew.-% Buten-1, 358 ppm n-Butan, 95 ppm trans-2-Buten, 112 ppm cis-2-Buten und 182 ppm Butadien eingesetzt. Der Rest sind im Wesentlichen als Verunreinigungen enthaltene C₃- und C₅-Kohlenwasserstoffe sowie weitere übliche Verunreinigungen, die bei der Herstellung von Butenen anfallen.

**Tabelle 1: Prozessparameter**

| | Anteil der Gasphase im Feedstrom [Gew.-%] | Verhältnis H₂/Butadien [mol/mol] | Druck am Reaktorausgang [bar(g)] | Temperatur am Reaktoreingang [°C] |
|---|---|---|---|---|
| Beispiel 1 | 15 | 4,7 | 3,17 | 40 |
| Beispiel 2 | 14 | 4,7 | 2,20 | 30 |
| Beispiel 3 | 15 | 2,1 | 3,17 | 40 |
| Beispiel 4 | 15 | 5,0 | 3,17 | 40 |
| Beispiel 5 | 15 | 3,1 | 3,17 | 40 |

**Tabelle 2: Zusammensetzung des Produktstroms**

| | Buten-1 [Gew.-%] | n-Butan [ppm] | trans-2-Buten [ppm] | cis-2-Buten [ppm] | Butadien [ppm] |
|---|---|---|---|---|---|
| Beispiel 1 | 99,73 | 441 | 705 | 668 | 2 |
| Beispiel 2 | 99,81 | 391 | 242 | 252 | 65 |
| Beispiel 3 | 99,78 | 435 | 435 | 362 | 30 |
| Beispiel 4 | 99,55 | 656 | 1524 | 1421 | nicht nachweisbar |
| Beispiel 5 | 99,78 | 396 | 473 | 435 | 10 |

Den Beispielen ist eindeutig zu entnehmen, dass durch die Einstellung des Anteils der Gasphase eine Selektivhydrierung durchgeführt werden kann, bei der das Butadien durch Hydrierung umgesetzt wird. Weiterhin ist ersichtlich, dass der Anteil an Buten-1 nur geringfügig abnimmt. Die größte Abnahme am Gehalt an Buten-1 ist bei einer erhöhten Wasserstoffzufuhr ersichtlich. Aber selbst hier nimmt der Gehalt an Buten-1 nur um 0,29 % ab, was zu einer Abnahme im Stoffstrom von 0,3 % führt. Durch Verkleinerung des Wasserstoffanteils lässt sich immer noch eine zufriedenstellende Abnahme des Gehalts an Butadien beobachten und eine noch weiter reduzierte Abnahme des Gehalts an Buten-1.

Für eine geforderte Spezifikation an Buten-1 von 99,5 Gew.-% konnte ein zufriedenstellender Produktstrom erhalten werden.

## Patentansprüche

1. Verfahren zur Entfernung von Dienen aus einem C₃- bis C₅-Kohlenwasserstoffe enthaltenden Stoffstrom durch Selektivhydrierung, folgende Schritte umfassend:
(a) Verdampfen eines Teils eines flüssigen C₃- bis C₅-Kohlenwasserstoffe enthaltenden Stoffstroms, Mischen eines gasförmigen und eines flüssigen C₃- bis C₅-Kohlenwasserstoffe enthaltenden Stoffstroms oder Kondensieren eines Teils eines gasförmigen C₃- bis C₅-Kohlenwasserstoffe enthaltenden Stoffstroms, so dass nach der Verdampfung 2 bis 50 mol-% des C₃- bis C₅-Kohlenwasserstoffe enthaltenden Stoffstrom in der Gasphase vorliegen,
(b) Zufuhr des teilverdampften C₃- bis C₅-Kohlenwasserstoffe enthaltenden Stoffstroms in eine einen Hydrierkatalysator enthaltende Reaktionsstufe und Zugabe von Wasserstoff in einem Anteil von 2 bis 20 Mol je Mol Dien, das in dem C₃- bis C₅-Kohlenwasserstoffe enthaltenden Stoffstrom enthalten ist, wobei die Zugabe des Wasserstoffs zusammen mit dem teilverdampften C₃- bis C₅-Kohlenwasserstoffe enthaltenden Stoffstrom erfolgen kann oder über eine separate Zugabestelle,
(c) Trennen von Gasphase und Flüssigphase und Entnahme der Flüssigphase als Produktstrom.

2. Verfahren gemäß Anspruch 1, **dadurch gekennzeichnet, dass** zur Einstellung der Menge des verdampften Teils des C₃- bis C₅-Kohlenwasserstoffe enthaltenden Stoffstroms ein Inertgas zugegeben wird.

3. Verfahren gemäß Anspruch 2, **dadurch gekennzeichnet, dass** das Inertgas ausgewählt ist aus der Gruppe bestehend aus Stickstoff, Methan, Kohlendioxid, Edelgasen und Mischungen daraus.

4. Verfahren gemäß einem der Ansprüche 1 bis 3, **dadurch gekennzeichnet, dass** die Schritte (a) und (b) in einem Apparat mit Verdampfungsteil und Reaktionsteil mit den Hydrierkatalysator enthaltendem Katalysatorbett durchgeführt werden, wobei der Verdampfungsteil so angeordnet ist, dass der C₃- bis C₅-Kohlenwasserstoffe enthaltenden Stoffstrom vor Eintritt in das Katalysatorbett so weit verdampft ist, dass 2 bis 50 mol-% des C₃- bis C₅-Kohlenwasserstoffe enthaltenden Stoffstrom in der Gasphase vorliegen.

5. Verfahren gemäß einem der Ansprüche 1 bis 4, **dadurch gekennzeichnet, dass** zum Verdampfen eines Teils des C₃- bis C₅-Kohlenwasserstoffe enthaltenden Stoffstroms in Schritt (a) der C₃- bis C₅-Kohlenwasserstoffe enthaltende Stoffstrom in zwei Ströme geteilt wird, einer der zwei Ströme vollständig verdampft wird und nach dem Verdampfen wieder mit dem zweiten der zwei Ströme vermischt wird.

6. Verfahren gemäß einem der Ansprüche 1 bis 4, **dadurch gekennzeichnet, dass** der C₃- bis C₅-Kohlenwasserstoffe enthaltende Stoffstrom bei erhöhtem Druck erwärmt und in den Reaktor, der bei niedrigerem Druck betrieben wird, entspannt wird.

7. Verfahren gemäß einem der Ansprüche 1 bis 5, **dadurch gekennzeichnet, dass** die Reaktionsstufe einen separaten Reaktor umfasst und die Verdampfung in Schritt (a) so gesteuert ist, dass beim Eintritt des C₃- bis C₅-Kohlenwasserstoffe enthaltenden Stoffstroms in den Reaktor ein weiterer Teil verdampft, so dass nach Eintritt des C₃- bis C₅-Kohlenwasserstoffe enthaltenden Stoffstroms in den Reaktor 2 bis 50 mol-% des C₃- bis C₅-Kohlenwasserstoffe enthaltenden Stoffstroms in der Gasphase vorliegen.

8. Verfahren gemäß einem der Ansprüche 1 bis 7, **dadurch gekennzeichnet, dass** der C₃- bis C₅-Kohlenwasserstoffe enthaltenden Stoffstrom mindestens 99 Gew.-% C₃- bis C₅-Alkene mit genau einer Doppelbindung enthält.

9. Verfahren gemäß einem der Ansprüche 1 bis 8, **dadurch gekennzeichnet, dass** der Produktstrom mindestens 99 Gew.-% C₃- bis C₅-Alkene mit genau einer Doppelbindung enthält.

10. Verfahren gemäß Anspruch 8 oder 9, **dadurch gekennzeichnet, dass** das C₃- bis C₅-Alken mit genau einer Doppelbindung Buten-1 ist.

11. Verfahren gemäß einem der Ansprüche 1 bis 10, **dadurch gekennzeichnet, dass** der Anteil der Diene in dem C₃- bis C₅-Kohlenwasserstoffe enthaltenden Stoffstrom kleiner als 1000 ppm ist.

12. Verfahren gemäß einem der Ansprüche 1 bis 11, **dadurch gekennzeichnet, dass** die in Schritt (c) erhaltene Gasphase abgekühlt wird, so dass in der Gasphase enthaltene C₃-bis C₅-Alkene mit genau einer Doppelbindung kondensieren und Entnahme der kondensierten C₃- bis C₅-Alkene mit genau einer Doppelbindung als Produkt.

## Claims

1. A process for removing dienes from a material stream comprising C₃ to C₅ hydrocarbons by selective hydrogenation, comprising the following steps:
(a) vaporizing part of a material stream comprising liquid C₃ to C₅ hydrocarbons, mixing a gaseous material stream comprising C₃ to C₅ hydrocarbons and a liquid one, or condensing part of a material stream comprising gaseous C₃ to C₅ hydrocarbons, with the result that 2 to 50 mol% of the material stream comprising C₃ to C₅ hydrocarbons is present in the gas phase after vaporization,
(b) supplying the partially vaporized material stream comprising C₃ to C₅ hydrocarbons to a reaction step comprising a hydrogenation catalyst and adding hydrogen in a proportion of 2 to 20 moles per mole of diene present in the material stream comprising C₃ to C₅ hydrocarbons, where the hydrogen may be added together with the partially vaporized material stream comprising C₃ to C₅ hydrocarbons or via a separate addition point,
(c) separating the gas phase and liquid phase and withdrawing the liquid phase as a product stream.

2. The process according to claim 1, wherein an inert gas is added to adjust the amount of the vaporized part of the material stream comprising C₃ to C₅ hydrocarbons.

3. The process according to claim 2, wherein the inert gas is selected from the group consisting of nitrogen, methane, carbon dioxide, noble gases, and mixtures thereof.

4. The process according to any of claims 1 to 3, wherein steps (a) and (b) are carried out in an apparatus having a vaporization part and a reaction part holding the catalyst bed comprising the hydrogenation catalyst, where the design of the vaporization part is such that, before entry into the catalyst bed, the material stream comprising C₃ to C₅ hydrocarbons is vaporized to an extent that 2 to 50 mol% of the material stream comprising C₃ to C₅ hydrocarbons is present in the gas phase.

5. The process according to any of claims 1 to 4, wherein vaporization of part of the material stream comprising C₃ to C₅ hydrocarbons in step (a) is done by splitting the material stream comprising C₃ to C₅ hydrocarbons into two streams, completely vaporizing one of the two streams, and mixing this again with the second of the two streams after vaporization.

6. The process according to any of claims 1 to 4, wherein the material stream comprising C₃ to C₅ hydrocarbons is heated at elevated pressure and then depressurized in the reactor, which is operated at lower pressure.

7. The process according to any of claims 1 to 5, wherein the reaction step comprises a separate reactor and the vaporization in step (a) is controlled such that, on entry of the material stream comprising C₃ to C₅ hydrocarbons into the reactor, a further part vaporizes, with the result that, after entry of the material stream comprising C₃ to C₅ hydrocarbons into the reactor, 2 to 50 mol% of the material stream comprising C₃ to C₅ hydrocarbons is present in the gas phase.

8. The process according to any of claims 1 to 7, wherein the material stream comprising C₃ to C₅ hydrocarbons comprises not less than 99% by weight of C₃ to C₅ alkenes having precisely one double bond.

9. The process according to any of claims 1 to 8, wherein the product stream comprises not less than 99% by weight of C₃ to C₅ alkenes having precisely one double bond.

10. The process according to claim 8 or 9, wherein the C₃ to C₅ alkene having precisely one double bond is but-1-ene.

11. The process according to any of claims 1 to 10, wherein the diene content in the material stream comprising C₃ to C₅ hydrocarbons is less than 1000 ppm.

12. The process according to any of claims 1 to 11, wherein the gas phase obtained in step (c) is cooled, resulting in condensation of C₃ to C₅ alkenes having precisely one double bond that are present in the gas phase and withdrawal of the condensed C₃ to C₅ alkenes having precisely one double bond as product.

## Revendications

1. Procédé d'élimination de diènes d'un flux contenant des hydrocarbures en C₃-C₅ par hydrogénation sélective, comprenant les étapes suivantes:
(a) l'évaporation d'une partie d'un flux liquide contenant des hydrocarbures en C₃-C₅, le mélange d'un flux gazeux et d'un flux liquide contenant des hydrocarbures en C₃-C₅ ou la condensation d'une partie d'un flux gazeux contenant des hydrocarbures en C₃-C₅, de telle sorte qu'après l'évaporation, 2 à 50 % en moles du flux contenant des hydrocarbures en C₃-C₅ soient présents dans la phase gazeuse,
(b) l'introduction du flux contenant des hydrocarbures en C₃-C₅ partiellement vaporisé dans une étape réactionnelle contenant un catalyseur d'hydrogénation et l'ajout d'hydrogène en une proportion de 2 à 20 moles par mole de diène contenu dans le flux contenant des hydrocarbures en C₃-C₅, l'hydrogène pouvant être ajouté conjointement avec le flux contenant des hydrocarbures en C₃-C₅ partiellement vaporisé ou par l'intermédiaire d'un point d'ajout distinct,
(c) la séparation de la phase gazeuse et de la phase liquide et l'élimination de la phase liquide sous forme de flux de produit.

2. Procédé selon la revendication 1, **caractérisé en ce qu'**un gaz inerte est ajouté pour ajuster la quantité de partie vaporisée du flux contenant des hydrocarbures en C₃-C₅.

3. Procédé selon la revendication 2, **caractérisé en ce que** le gaz inerte est choisi dans le groupe constitué par l'azote, du méthane, le dioxyde de carbone, les gaz rares et de mélanges de ceux-ci.

4. Procédé selon l'une quelconque des revendications 1 à 3, **caractérisé en ce que** les étapes (a) et (b) sont réalisées dans un appareil comportant une partie évaporation et une partie réaction comportant un lit de catalyseur contenant le catalyseur d'hydrogénation, la partie évaporation étant agencée de telle sorte que le flux contenant des hydrocarbures en C₃-C₅ soit évaporé à tel point qu'avant d'entrer dans le lit de catalyseur, 2 à 50 % en moles du flux contenant des hydrocarbures en C₃-C₅ sont présents dans la phase gazeuse.

5. Procédé selon l'une quelconque des revendications 1 à 4, **caractérisé en ce que**, pour évaporer une partie du flux contenant des hydrocarbures en C₃-C₅ dans l'étape (a), le flux contenant des hydrocarbures en C₃-C₅ est scindé en deux flux, l'un des deux flux étant complètement évaporé et, après évaporation, de nouveau mélangé avec le second des deux flux.

6. Procédé selon l'une quelconque des revendications 1 à 4, **caractérisé en ce que** le flux contenant des hydrocarbures en C₃-C₅ est chauffé à haute pression et détendu dans le réacteur qui fonctionne à pression plus basse.

7. Procédé selon l'une quelconque des revendications 1 à 5, **caractérisé en ce que** l'étape réactionnelle comprend un réacteur distinct et **en ce que** l'évaporation dans l'étape (a) est contrôlée de telle sorte que, lorsque le flux contenant des hydrocarbures en C₃-C₅ entre dans le réacteur, une autre partie s'évapore, de façon qu'après l'entrée du flux contenant des hydrocarbures en C₃-C₅ dans le réacteur, 2 à 50 % en moles du flux contenant des hydrocarbures en C₃-C₅ soient présents dans la phase gazeuse.

8. Procédé selon l'une quelconque des revendications 1 à 7, **caractérisé en ce que** le flux contenant des hydrocarbures en C₃-C₅ contient au moins 99 % en poids d'alcènes en C₃-C₅ comportant exactement une double liaison.

9. Procédé selon l'une quelconque des revendications 1 à 8, **caractérisé en ce que** le flux de produit contient au moins 99 % en poids d'alcènes en C₃-C₅ comportant exactement une double liaison.

10. Procédé selon la revendication 8 ou 9, **caractérisé en ce que** l'alcène en C₃-C₅ comportant exactement une double liaison est le but-1-ène.

11. Procédé selon l'une quelconque des revendications 1 à 10, **caractérisé en ce que** la proportion de diènes dans le flux contenant des hydrocarbures en C₃-C₅ est inférieure à 1 000 ppm.

12. Procédé selon l'une quelconque des revendications 1 à 11, **caractérisé en ce que** la phase gazeuse obtenue dans l'étape (c) est refroidie de telle sorte que des alcènes en C₃-C₅ comportant exactement une double liaison contenus dans la phase gazeuse se condensent et que les alcènes en C₃-C₅ condensés comportant exactement une double liaison soient prélevés comme produit.
